Europäisches Patentamt

European Patent Office

Office européen des brevets

(11) Veröffentlichungsnummer: **0 519 273 A2**

## (12) EUROPÄISCHE PATENTANMELDUNG

(21) Anmeldenummer: **92109364.7**

(22) Anmeldetag: **03.06.92**

(51) Int. Cl.5: **G01N 7/14**

(30) Priorität: **18.06.91 DE 4119966**

(43) Veröffentlichungstag der Anmeldung:
**23.12.92 Patentblatt 92/52**

(84) Benannte Vertragsstaaten:
**AT CH DE FR GB IT LI**

(71) Anmelder: **SPÜHL AG**
**Lukasstrasse 18**
**CH-9009 St. Gallen(CH)**

(72) Erfinder: **Strub, Fritz, Dipl. Ing.**
**Lehnstrasse 18**
**W-9014 St.Gallen(DE)**

(74) Vertreter: **Riebling, Peter, Dr.-Ing.,**
**Patentanwalt**
**Rennerle 10, Postfach 31 60**
**W-8990 Lindau/B.(DE)**

(54) Messeinrichtung zur Erfassung der Gasbeladung einer Kunststoffkomponente.

(57) Zum Messen der Luftbeladung einer Kunststoffkomponente für eine Niederdruck-Mehrkomponenten-Schäumanlage wird die gasbeladene Kunststoffkomponente in eine Messkammer eingelassen. Der Gasdruck der Kunststoffkomponente verschiebt einen Meßkolben in der Meßkammer und der Verschiebungsweg des Kolbens ist ein Maß für die Gasbeladung.

EP 0 519 273 A2

Die Erfindung betrifft eine Meßeinrichtung mit den Merkmalen des Oberbegriffs von Patentanspruch 1.

Bei derartigen Schäumanlagen ist es bekannt, daß eine A-Komponente (z.B. Polyol) unter geregeltem Druck in einem Komponentenbehälter aufbewahrt wird.

Es ist eine Zweite Komponente (die B-Komponente) vorhanden, die einen gleichen Kreislauf aufweist wie es für die A-Komponente nachfolgend beschrieben wird. Beide Komponenten werden in einem Mischkopf Zusammengeführt und dann bei einem Schuß über einen Schußkopf auf ein Werkstück aufgebracht, wobei die in der Komponente A enthaltene Luft maßgebend für die Porösität des gespritzten Kunststoffgegenstandes ist. Ggf. kann auch die Komponente B ungewollt Luft enthalten, die dann auch einen Einfluß auf den Schaum haben kann.

Ein derartiger Kunststoffgegenstand ist bevorzugt eine Dichtung, die mit der eingangs genannten Anlage auf ein Werkstück aufgespritzt wird und nachfolgend dann chemisch reagiert, wobei die Dichtung aufschäumt und sich dann verfestigt.

Bisher bestand das Problem, daß der Luftanteil in der A-Komponente nur schwierig gleichmäßig Zu halten war, denn es war bisher bekannt, diese Luft in den A-Komponentenbehälter einzublasen, wobei sie sich unter Wirkung eines Rührwerkes möglichst innig mit dieser A-Komponente vereinigen sollte.

Nachdem der Behälterinnenraum des Komponentenbehälters unter Druck steht und die Luft unter höherem Druck feinperlig in die A-Komponente eingeführt wird, kommt es zu einer Bindung der Luft an die A-Komponente. Dieses mit Luft versetzte A-Komponentengemisch wird über einen Materialauslaß und eine Dosierpumpe dem Mischkopf zugeführt.

Es ist ebenso bekannt, der B-Komponente einen gleichen Kreislauf, bestehend aus Komponentenbehälter, Materialauslaß, Dosierpumpe und Zulauf in den Mischkopf, zuzuordnen, wobei der B-Komponente keine Luft Zugeführt wird, aber Luft enthalten kann.

Wie eingangs bereits erwähnt, war es bisher schwierig, die Luftmenge in den Komponentenbehälter so Zu regeln, daß immer, unabhängig vom Füllgrad des Komponentenbehälters, ein gleichmäßiger Prozentanteil der Luft in der A-Komponente beibehalten wurde.

Bisher ist es bekannt, die Menge der Luft in der A-Komponente durch eine Meßeinrichtung zu erfassen, die im Auslauf des A-Komponentenbehälters angeordnet war.

Das Meßprinzip dieser Meßeinrichtung beruhte darauf, daß die zu messende Flüssigkeit in eine Meßkammer eingeleitet wurde, in welcher Meßkammer ein Schallgeber vorhanden war und ein Schallempfänger. Durch die Schallbeaufschlagung des Meßvolumens wurde durch die in der A-Komponente enthaltende Luft eine gewiße Dämpfung der Schallwellen erzeugt, welche von dem Schallempfänger erfaßt wurde. Durch die Auswertung der Dämpfung konnte somit der Anteil der Luft in der A-Komponete bestimmt werden. Nachteil der bekannten Meßeinrichtung ist allerdings, daß der Meßaufwand unverhältnismäßig groß ist und eine derartige Vorrichtung sehr teuer in der Herstellung und Wartung ist.

Der vorliegenden Erfindung liegt deshalb die Aufgabe zugrunde, eine Meßvorrichtung der eingangs genannten Art so weiterzubilden, daß sie wesentlich kostengünstiger herstellbar ist, betriebssicherer arbeitet und einen schnellen Überblick über die in der A-Komponente enthaltende Luft gewährleistet.

Die Lösung dieser Aufgabe erfolgt durch die kennzeichnenden Maßnahmen von Patentanspruch 1. Demnach ist es wesentlich, daß die Meßvorrichtung in der Auslaufleitung des Komponentenbehälters angeordnet ist und ein Ventil die Meßkammer öffnet und schließt, wobei die Meßkammer aus einem Meßkolben besteht, der als Verdrängungskolben in einem Meßraum verschiebbar angeordnet ist, und daß der Verschiebungsweg des Meßkolbens in der Meßkammer durch eine Wegmessung erfaßt wird.

Wesentliches Merkmal der vorliegenden Erfindung ist also, daß die Meßvorrichtung in der Auslaufleitung des Komponentenbehälters angeordnet ist und daß mit Hilfe eines Ventils eine genau festgelegte Charge der mit Luft beladenen Komponente in einem Meßraum einleitbar ist. Sobald der Meßraum gefüllt ist wird er gegen weiteres Eindringen der mit Luft beladenen Komponente geschlossen und die Messung beginnt.

Vor Beginn der Messung wurde der Meßkolben mit Hilfe einer Gegenkraft (z. B. einer Feder oder eines Elektromagneten) in angehobener Stellung in seiner Verdrängungsposition in dem Meßraum gehalten.

Bei einer Konstruktion mit einer Rollmembrane muß das Anheben des Kolbens durch Luftdruck erfolgen, damit die Membrane nicht umklappen kann. Die Feder zieht nach unten, so daß der Druck unter dem Kolben immer höher ist als über der Rollmembrane. (Ausnahme: unterste Extremstellung, wo die Membrane gestützt wird).

Die Kräfte zum Heben des Kolbens sind sehr hoch, z.B. bei 5 bar Komponentendruck: ca. 700 N. Dies ist mit einem Elektromagneten kaum möglich.

Nach Beginn der Messung wird diese Gegenkraft entfernt und der Kolben wird durch die in den Meßraum eindringende, luftbeladene Komponenten nach unten verdrängt.

Die Gegenkraft nach oben muß langsam vermindert werden, bis der Kolben langsam nach unten zu laufen beginnt. Der Druck darf in der A-Komponente nicht abfallen, bis das Kugelventil schließt.

2

(Strömungswiderstände der A-Komponente).

Nachdem die luftbeladene A-Komponente mit einem Druck von im Bereich 2 bis 10 bar in der Auslaßleitung fließt, wird unter diesem Druck auch der Meßraum mit dieser luftbeladenen Komponente gefüllt und nachfolgend durch das Ventil von oben her abgeschlossen, so daß keine weitere Komponente mehr nachfließen kann.

Durch das Schließen des Ventils und durch Verdrängen des Meßkolbens nach unten wird eine Druckentlastung in dem Meßraum ausgeführt, wodurch der Druck von zuerst im Bereich 2 bis 10 bar auf einen wesentlich geringeren Wert absinkt. Durch diese Expansion im Meßraum beginnt die A-Komponente auszugasen und gibt in Form von Luftperlen die Luft in den Meßraum ab, wodurch eine weitere Verdrängung des Meßkolbens nach unten in Richtung seiner Längsachse erfolgt. Diese Verdrängung des Kolbens aufgrund der ausgasenden Luft wird für die Messung des Luftinhaltes des Meßraumes verwendet.

Der weitere Verschiebungsweg des Kolbens aufgrund des Ausgasens der A-Komponente wird durch eine Wegmessung des Kolbens erfaßt. Hierbei wird es bevorzugt, wenn die Längsverschiebung des Meßkolbens durch einen elektrischen Näherungsschalter erfaßt wird. Hierbei kann es vorgesehen sein, daß am unteren Ende der Kolbenstange des Meßkolbens ein Meßdorn angebracht ist, dem ein Näherungsschalter gegenüberliegt. Der Meßdorn nähert sich also mit zunehmender Entgasung der A-Komponente den im Bodenbereich der Meßvorrichtung angeordneten Näherungsschalter. Die Annäherung dieses Meßdornes an den Näherungsschalter ist ein Maß für den Luftinhalt der in der Meßkammer angeordneten Flüssigkeit.

Ist die Flüssigkeit nicht mit Luft beladen, dann kommt es zu keiner Annäherung des Meßdornes an den Näherungsschalter und dies wird als Signal abgeleitet, um mehr Luft in den Komponentenbehälter einzubringen. Sobald dann eine nächste Charge in die Meßkammer für eine nächste Messung eingeleitet wird, kommt es dann zu einer Ausgasung der Luft in der Meßkammer und der Kolben wird aufgrund dieser Volumenzunahme nach unten verdrängt und der Meßdorn nähert sich somit dem Näherungsschalter. Die Annäherung des Meßdornes an den Näherungsschalter ist also direkt proportional zu der Luftmenge, welche im Meßraum der angeordneten A-Komponente enthalten ist.

Auf diese Weise kann relativ genau der Luftinhalt der A-Komponente bestimmt werden, und zwar durch kontinuierlich nacheinander folgenden Messungen, wobei in nacheinander folgenden Meßzyklen immer eine bestimmte Charge aus der A-Komponente entnommen wird.

Eine derartige Vorrichtung ist sehr kostengünstig, denn man benötigt keine elektronischen Schallwandler und Schallgeber, sondern man benötigt nur einen einfachen Meßkolben in Verbindung mit einem Meßraum, der nach unten über eine Topfmembrane abgedichtet ist.

Um den Meßkolben in seiner angehobenen Nicht-Meß-Stellung zu halten wird es hierbei bevorzugt, wenn der Meßkolben von unten her über Druckluft in seine angehobene Stellung in dem Meßraum gehalten wird, um somit den Meßraum durch Verdrängung von der Komponente freizumachen.

Ferner wird es bevorzugt, wenn mit dem Meßkolben selbst auch das Ventil direkt betätigt wird, welches ein Einlaufen der luftbeladenen A-Komponente in den Meßraum bewerkstelligt. Hierbei ergibt sich eine besonders einfache Konstruktion, denn in der angehobenen Verdrängungsstellung des Meßkolbens wird über eine Kolbenstange des Meßkolbens ein Kugelventil derart geschlossen, daß die A-Komponente zwar über eine Durchflußbohrung in die Meßvorrichtung ein- und wieder ausfließen kann, daß aber aufgrund der geschlossenen Stellung dieses Kugelventils die A-Komponente nicht in den Meßraum einfließen kann.

Sobald die Gegenkraft von dem Meßkolben entfernt wird, wird somit der Kolben aufgrund des bestehenden Druckes von oben her (von der A-Komponente) nach unten verdrängt, wobei gleichzeitig das Kugelventil geöffnet wird und die Flüssigkeit in den Meßraum einfließt. Gleichzeitig wird das Kugelventil mitgenommen und kommt dann auf einem unteren Ventilsitz zur Anlage, wodurch das weitere Einfließen der Flüssigkeit in den Meßraum verhindert wird. Der Meßraum ist somit mit luftbeladener Flüssigkeit gefüllt und die eigentliche Messung der darin enthaltenden Luft kann beginnen.

In der oberen Stellung ist kein Ventilsitz für die Kugel vorhanden
Nachdem der Meßraum von oben her durch das Kugelventil geschlossen wird und der Meßkolben nach unten ausweicht, erfolgt somit ein Druckabfall in dem Meßraum, wodurch es zu der vorher beschriebenen Entgasung der Flüssigkeit kommt und das Volumen in dem Meßraum nimmt weiter zu, so daß der Meßkolben nach unten verdrängt wird und hierdurch der Näherungsschalter anspricht.

Das beschriebene Meßprinzip kann in verschiedenen Ausführungen abgewandelt werden.

In einer ersten Ausführungsform kann es vorgesehen sein, daß das vorher beschriebene, vom Meßkolben direkt betätigte Kugelventil durch eine eigene Steuerung betätigt werden kann.

Eine derartige Steuerung kann entweder eine Elektromagnetsteuerung oder eine Druckluftsteuerung sein.

In einer weiteren Ausbildungsform kann es vorgesehen sein, daß die angehobene Stellung des Meßkolbens nicht durch Druckluft erfolgt, sondern daß stattdessen ein Elektromagnet diese Gegenkraft erzeugt.

In diesem Falle könnte keine (fast reibungsfreie) Rollmembrane verwendet werden. Die Rollmembrane würde umklappen (Beschädigung der Membrane sowie Volumenfehler).

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander. Alle in den Unterlagen - einschließlich der Zusammenfassung - offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technki neu sind.

Im folgenden wird die Erfindung anhand von lediglich einen Ausführungsweg darstellende Zeichnungen näher erläutert. Hierbei gehen aus den Zeichnungen und ihrer Beschreibung weitere erfindungswesentliche Merkmale und Vorteile der Erfindung hervor.

Es zeigen:

Figur 1: schematisiert ein Kreislaufbild für die A-Komponente;

Figur 2: die Meßvorrichtung im Schnitt in der Nicht-Meß-Stellung;

Figur 3: die Meßvorrichtung nach Figur 2 in der Meßstellung.

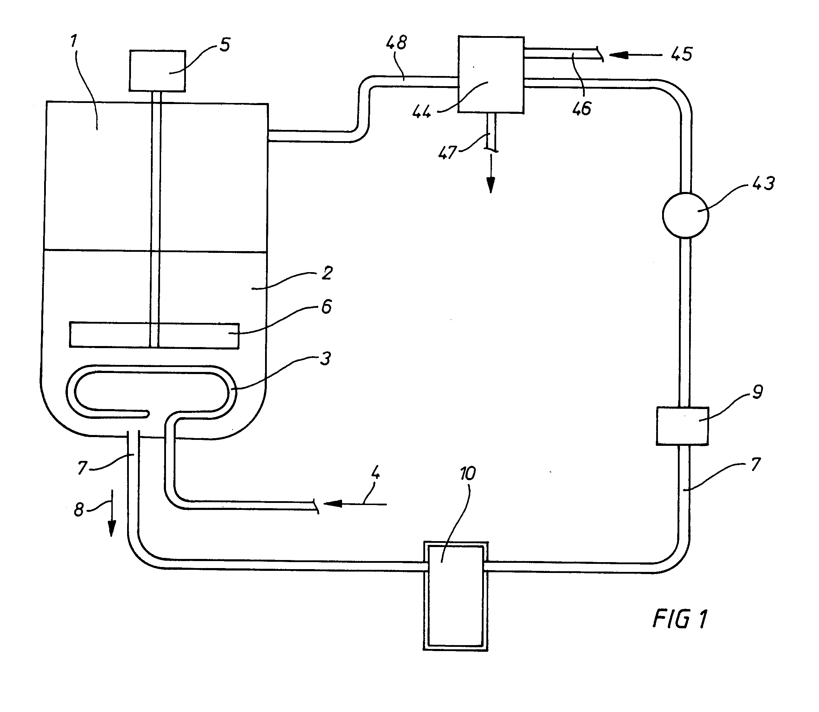

Gemäß Figur 1 ist in einem Komponentenbehälter 1 eine sogenannte A-Komponente enthalten, die z. B. aus dem chemischen Stoff Polyol (eingetragenes Warenzeichen der Firma DuPont) besteht. Polyol ist ein Polyisocyanat.

In dem Komponentenbehälter 1 ist also die A-Komponente als Material 2 enthalten und wird von einem Rührwerk 6 umgerührt.

Der Innenraum des Komponentenbehälters 1 wird ständig unter einem bestimmten Druck gehalten. Dieser Druck kann im Bereich von 2 bis 10 bar liegen; bevorzugt liegt er bei 6 bar.

Unterhalb des Rührwerkes 6 wird in Pfeilrichtung 4 Luft über einen Luftverteiler 3 in den Komponentenbehälter 1 eingeblasen, wobei die Luft feinperlig über entsprechende Bohrungen des Luftverteilers 3 in das Material 2 hineindiffundiert.

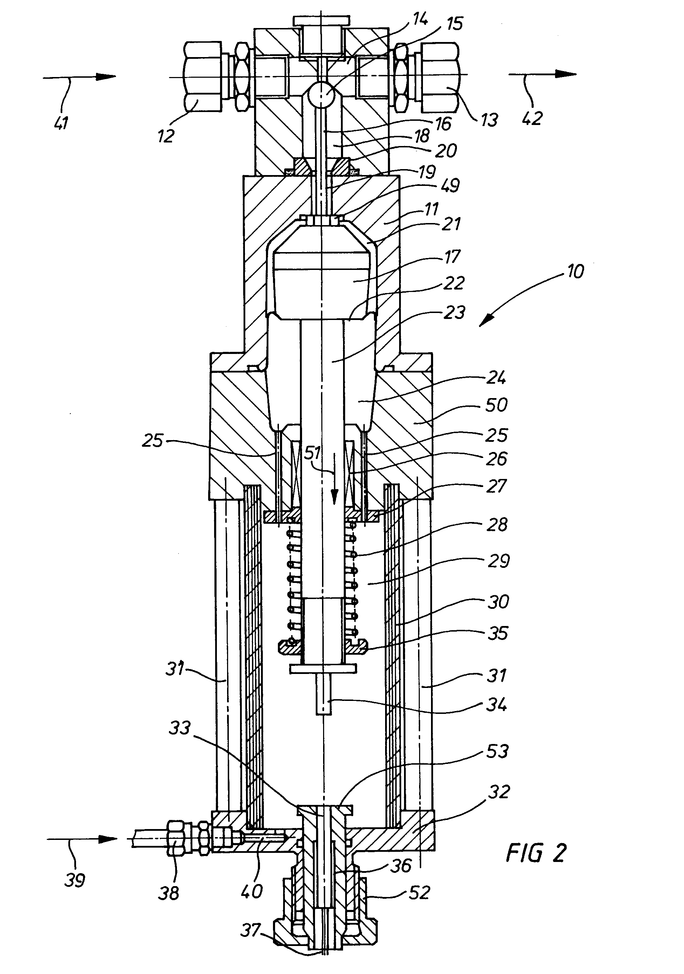

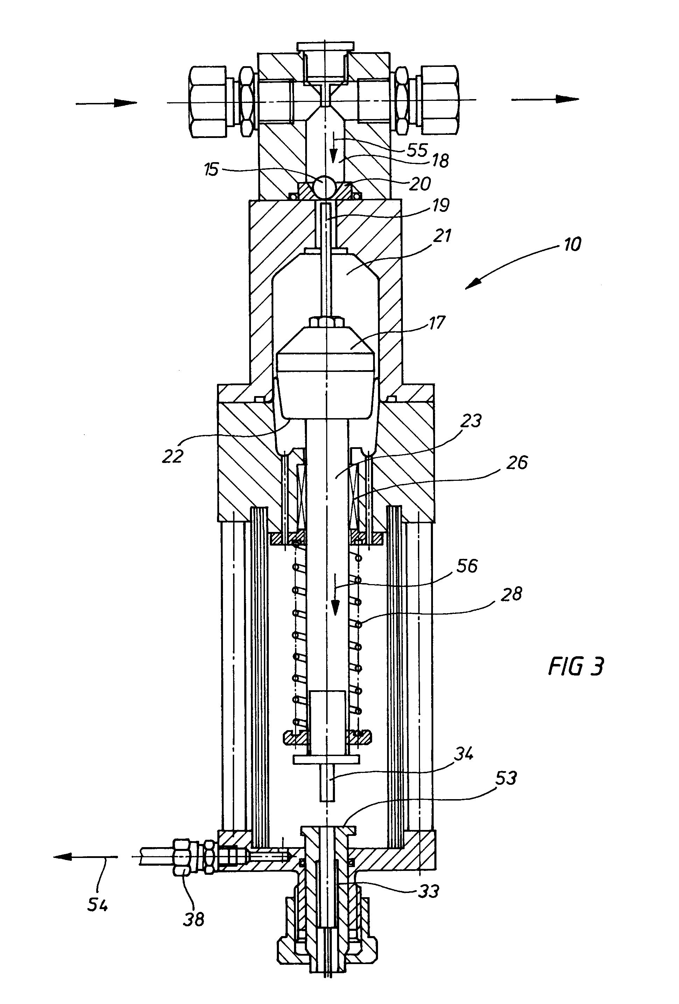

Die so mit Luft beladene Komponente wird über einen Auslaß 7 in Pfeilrichtung 8 einer Meßvorrichtung 10 zugeführt. Die Meßvorrichtung wird später anhand der Figuren 2 und 3 erläutert.

Im Anschluß an die Meßvorrichtung ist gegebenenfalls ein Filter 9 in der Leitung angeordnet, hinter dem eine Dosierpumpe 43 angeordnet ist.

Die Dosierpumpe 43 fördert die luftbeladene A-Komponete in einen Mischkopf 44, wo gleichzeitig in Pfeilrichtung 45 eine B-Komponente 46 eingebracht wird. Wie bereits schon eingangs erwähnt, hat die B-Komponente den gleichen Kreislauf wie in Figur 1 erläutert, mit Ausnahme jedoch, daß keine Luft eingeblasen wird.

Am Mischkopf 44 ist ein Schußkopf 47 angeordnet, über den die vermischte AB-Komponente auf ein Werkstück aufgebracht wird.

Am Ausgang des Mischkopfes 44 ist eine Rezirkulationsleitung 48 angeordnet, über welche die nicht verbrauchte A-Komponente wieder in den Komponentenbehälter 1 zurückgeführt wird.

Problem ist nun, daß man nun eine relativ gleichmäßige Luftbeladung der A-Komponente erreichen will, um ein möglichst gleichmäßiges Aufschäumen der Mischkomponenten A + B im Schußkopf 47 auf dem Werkstück zu gewährleisten.

Hierzu muß die Einbringung von Luft in Pfeilrichtung 4 geregelt werden.

Die Meßvorrichtung 10 dient zur Erfassung der Menge der Luftbeladung der A-Komponente.

Die A-Komponente wird in Pfeilrichtung 41 über einen Einlaßstutzen 12 in die Meßvorrichtung 10 eingelassen und verläßt im Durchflußbetrieb über eine Durchflußbohrung 14 den Auslaßstutzen 13 in Pfeilrichtung 42. (Vgl. Figur 2).

In dieser Nicht-Meß-Stellung ist also die Meßvorrichtung gemäß Figur 2 nicht in Betrieb.

Die Meßvorrichtung besteht im wesentlichen aus einem Grundkörper 11, wobei von der Durchflußbohrung 14 abzweigend ein unterer Kanal 18 vorhanden ist.

In der Offenstellung wird hierbei ein Kugelventil 15 durch eine Kolbenstange 16 eines Meßkolbens 17 in der Offenstellung gehalten, so daß das Material der A-Komponente noch in den Kanal 18 einströmt und ebenso in einem darunterliegenden Kanal 19.

Ein Ventilsitz ist nicht vorhanden. Dieser würde stören. Es ist immer ein Spalt 49 von ca. 2 mm vorhanden. Der Weg des Kolbens nach oben ist über den Anschlag der Kugel beschränkt.

An der anderen Seite wird der Meßraum 21 durch eine Topfmembrane 22 begrenzt, die abdichtend in entsprechenden gehäusefesten Ringnuten zwischen dem Grundkörper 11 und dem Oberteil 50 gehalten wird.

An der Unterseite der Topfmembrane 22 ist eine Zylinderbohrung 24 angeordnet, welche über Kanäle 25 luftleitend mit einer unteren Bohrung 29 verbunden ist.

An der Unterseite des Meßkolbens 17 ist eine Kolbenstange 23 befestigt, welche über Längskugellager 26 verschiebbar in dem Oberteil 50 gehalten ist. Die Längskugellager 26 sorgen für eine leichte Verschiebbarkeit der Kolbenstange 23 in dem Oberteil 50.

Eine Feder 28 ist vorhanden, die sich mit ihrer einen Seite an einem gehäusefesten Ring 27 abstützt und mit ihrer anderen Seite an einem Ring 35, der auf der Kolbenstange 23 mit einem Gewinde aufschraubbar befestigt ist. Die Feder 28 dient nicht zur Überwindung der Reibung (diese ist unbedeutend klein). Die Feder 28 erzeugt einen Unterdruck im Messraum von z.B. ca. 0,5 bar. Der Unterdruck fördert im Messraum die Gasentbindung, erhöht das Volumen der Bläschen und vergrößert damit den Messausschlag des Kolbens. Der Unterdruck ist mit dem Ring 35 einstellbar.

Die Feder 28 ist sehr stark, z.B. ca. 90 N. Sie hält auch die Membrane immer gespannt. Daher gibt es keinen Volumenfehler.

Auf diese Weise kann durch Verdrehung des Ringes 35 die Vorspannung der Feder 28 eingestellt werden.

An der Unterseite der Kolbenstange 23 ist ein Meßdorn 34 angebracht, dem gehäusefest gegenüberliegend in einem Bodenkörper 32 der Meßvorrichtung ein Näherungsschalter 33 gegenüberliegt. Dieser Näherungsschalter 33 ist an sich bekannt und arbeitet bevorzugt nach einem elektronischen Meßprinzip.

Wichtig hierbei ist, daß der Näherungsschalter 33 in Richtung seiner axialen Längserstreckung mit Hilfe einer Überwurfmutter 52 verstellbar und feststellbar ist, so daß die Meßfläche 53 des Näherungsschalters 33 in einem gewißen Bereich in die Bohrung 29 hinein- und herausverstellbar ist.

Auf diese Weise kann der Näherungsschalter 33 geeicht werden, wie anhand der Figur 3 noch später erläutert werden wird.

Über einen Einlaß 38 wird ferner in Pfeilrichtung 39 Druckluft über die Bohrung 40 in die Bohrung 29 eingelassen, wobei diese Druckluft einen höheren Druck aufweist, als der Druck der A-Komponente.

Auf diese Weise wird der Meßkolben 17 in seiner angehobenen Position in der Meßkammer 21 gehalten. Der Ventilsitz 20 ist geöffnet.

Damit man die gesamte Meßvorrichtung von außen beobachten kann, wird es bevorzugt, wenn die Wandung 30 aus einem durchsichtigen Kunststoffmaterial ist und zur einfachen Zerlegbarkeit der gesamten Vorrichtung wird es ferner bevorzugt, wenn der Bodenkörper 32 über entsprechende Schrauben 31 mit dem Oberteil 50 verbunden ist.

Ein Meßzyklus gemäß Figur 3 wird nun wie folgt eingeleitet.

Die Druckluft, die vorher in Pfeilrichtung 39 in die Bohrung 29 eingeleitet wurde, wird nun entfernt (Pfeilrichtung 54), so daß nun die Gegenkraft auf die Topfmembrane 22 entfällt und die A-Komponente in Pfeilrichtung 55 über den Kanal 18 und den noch geöffneten Ventilsitz 20 in den Meßraum 21 einströmt, wobei gleichzeitig der Meßkolben 17 in Pfeilrichtung 55 nach unten verdrängt wird. Hierbei wird die Feder 28, die eine relativ großeFederkraft aufweist, entspannt. Sie sorgt für ein langsames Senken bis die Kugel schließt.

Im einzelnen wird also die Gegenkraft auf die Topf- bzw. Rollmembran langsam durch Abblasen der Luft vermindert. Der Kolben senkt sich, wobei der Druck im Meßraum konstant bleibt.

Sobald die Kolbenstange 19 unterhalb des Ventilsitzes 20 zu liegen kommt, dichtet das Kugelventil 15 den Ventilsitz 20 ab, so daß keine weitere Komponente mehr in Pfeilrichtung 55 in den Meßraum 21 einfließen kann. Der Meßraum 21 ist damit mit einer genau vorgegebenen Charge der luftbeladenen Flüssigkeit gefüllt.

Aufgrund der Tatsache, daß nun der Gegendruck auf die Topfmembrane 22 entfallen ist, kommt es zu einer starken Druckentspannung im Meßraum 21 und die darin enthaltene luftbeladene Flüssigkeit beginnt zu entgasen, nämlich man erreicht eine Druckentspannung auf unter 1 bar absolut: Auf ca. 0,5 bar (Bewegung mit Hilfe der Federkraft nach unten).

Der in der in Figur 3 dargestellten Verschiebungslage eingezeige Ventilkolben wird somit aufgrund des zunehmenden Luftvolumens noch weiter in Pfeilrichtung 56 nach unten verdrängt, wodurch sich der Meßdorn 37 der Meßfläche 53 des Näherungsschalters 33 nähert.

Nach einer gewißen Meßzeit, die im Sekundenbereich liegt, erfolgt keine weitere Entgasung mehr der luftbeladenen Flüssigkeit im Meßraum 21, so daß der Meßdorn 34 seine endgültige Position gegenüber der Meßfläche 53 eingenommen hat. Diese Position wird nun meßtechnisch über den Näherungsschalter 33 erfaßt und dieses Signal wird angezeigt, so daß man hieraus eine Anzeige für die Luftbeladung der A-Komponente erhält.

Dieses Signal kann selbstverständlich später auch für die Regelung der Einführung der Luft in Pfeilrichtung 4 in den Luftverteiler 3 verwendet werden, wobei eine Regelstrecke in an sich bekannter Weise vorgesehen sein kann.

Es ergibt sich dann dadurch ein geschlossener Regelkreis.

Es ist ein Näherungsschalter vorgesehen. Es wird also kein Abstand gemessen mit dem Signal "AUF-ZU" (bei z.B. ca. 1 mm Annäherung). Die Einstellung des Schaltpunktes erfolgt mit der Mutter 52.

Der Messdorn 23 ist zur Verminderung von Beschädigungen des Näherungsschalters und evtl. der Rollmembrane in der Kolbenstange 23 versenkbar.

Ein Regelkreis ist nur bei laufender Wiederholung der Messung möglich. Ein Schutz gegen zu wenig Luft, aber ebenfalls gegen zu viel Luft, ist wichtig.

Beispielsweise wird eine chargenweise Messung so durchgeführt, daß man erst eine geringe Luftbeladung des Materials 2 vornimmt und dann eine erste Messung durchführt, wobei man dann in der Regel erkennt, daß der Meßdorn 34 sich nicht genügend der Meßfläche 53 des Näherungsschalters 33 nähert, so daß der Näherungsschalter 33 kein Meßsignal abgibt. Erst wenn man dann noch weitere Luft in Pfeilrichtung 4 in den Luftverteiler 3 einführt kommt es zu einer erhöhten Luftbeladung der A-Komponente und bei der nächsten Messung der nächsten Charge wird dann eine stärkere Ausgasung der luftbeladenen Komponente im Meßraum 21 stattfinden und der Meßdorn 34 wird sich auf eine nähere Entfernung der Meßfläche 53 nähern, so daß jetzt der Näherungsschalter 33 anspricht und anzeigt, daß eine genügende Luftbeladung erreicht wurde.

Bei einer zu großen Beladung der A-Komponente mit Luft kann noch ein Grenzwertschalter vorhanden sein, der diese übermäßige Luftbeladung (maximalen Ausschlag der Kolbenstange 23 in Pfeilrichtung 56) anzeigt und dann können entsprechende Gegenmaßnahmen eingeleitet werden, um diese übermäßige Luftbeladung zu verhindern. Da Material verbraucht wird, sinkt bei abgestellter Luftbeladung der Luftgehalt von selbst wieder ab.

Die Messung muß in genügend kurzen Zeitabständen erfolgen, so daß keine Überbeladung entstehen kann.

Wichtig hierbei ist, daß es sich um eine diskontinuierliche Messung, d.h. also eine chargenweise Messung der luftbeladenen A-Komponente handelt, wobei sehr schnell hintereinander derartige Messungen vorgenommen werden können.

Die vorliegende Meßvorrichtung ist sehr empfindlich und kann auch kleinste, ausgasende Luftmengen im Meßraum 21 erfassen. Zur Steigerung der Empfindlichkeit ist die vorher beschriebene Feder 28 vorgesehen. Die Feder 28 ist stark dimensioniert und steigert daher die Empfindlichkeit.

Die Gasentbindung braucht nämlich etwas Zeit und den Anstoss durch den Unterdruck.

Nach Vollendung der Messung nach Figur 3 wird ein neuer Meßzyklus dadurch eingeleitet, indem die Druckluft wiederum in Pfeilrichtung 39 auf den Einlaß 38 gegeben wird.

Der Meßkolben 17 wird hiermit in Gegenrichtung zur eingezeichneten Pfeilrichtung 56 nach oben verdrängt und verdrängt die Komponente aus dem Meßraum 21 über das sich jetzt öffnende Kugelventil 15 wiederum in den Kanal 18 hinein, wo es über die Durchflußbohrung 14 wieder in den Kreislauf eingespeist wird.

EP 0 519 273 A2

## ZEICHNUNGS-LEGENDE

1 Komponentenbehälter
2 Material
3 Luftverteiler
4 Pfeilrichtung
5 Motor
6 Rührwerk
7 Auslaß
8 Pfeilrichtung
9 Filter
10 Meßvorrichtung
11 Grundkörper
12 Einlaßstutzen
13 Auslaßstutzen
14 Durchflußbohrung
15 Kugelventil
16 Kolbenstange
17 Meßkolben
18 Kanal
19 Kanal
20 Ventilsitz
21 Meßraum
22 Topfmembran
23 Kolbenstange
24 Zylinderbohrung
25 Kanal

26 Längskugellager
27 Ring
28 Feder
29 Bohrung
30 Wandung
31 Schraube
32 Bodenkörper
33 Näherungsschalter
34 Meßdorn
35 Ring
36 Gewinde
37 Anschluß
38 Einlaß
39 Pfeilrichtung
40 Bohrung
41 Pfeilrichtung
42 Pfeilrichtung
43 Dosierpumpe
44 Mischkopf
45 Pfeilrichtung
46 B-Komponente
47 Schußkopf
48 Rezirkulationsleitung
49 Ventilsitz
50 Oberteil
51 Pfeilrichtung
52 Überwurfmutter
53 Meßfläche
54 Pfeilrichtung
55 "
56 "

**Patentansprüche**

1. Meßeinrichtung zur Erfassung der Gasbeladung (Luftbeladung) einer Kunststoffkomponente für eine Niederdruck-Mehrkomponenten-Schäumanlage, die im Auslauf eines Behälters für diese gasbeladene Kunststoffkomponente angeordnet ist, **dadurch gekennzeichnet**, daß eine Meßkammer (21) vorgese-

7

hen ist, die über ein Ventil (15) mit der Komponente (2) gefüllt werden kann, deren Gasbeladung gemessen werden soll, daß eine Wand der Kammer (21) als Kolben (17) ausgebildet ist, und daß eine Einrichtung (33) vorgesehen ist, die den Verschiebungsweg des Kolbens (17) unter dem Einfluß des in der Komponente gelösten Gases mißt.

2. Meßeinrichtung nach Anspruch 1, **dadurch gekennzeichnet**, daß eine Gegenkraft vorgesehen ist, die den Meßkolben (21) in seine Ausgangsstellung zurückführt.

3. Meßeinrichtung nach Anspruch 1 oder 2, **dadurch gekennzeichnet**, daß im Verschiebungsweg des Kolbens (17) ein Näherungsschalter (33) vorgesehen ist und daß mit dem Kolben (17) ein Meßdorn (34) verbunden ist, der in der Nähe des Näherungsschalters verschiebbar ist.

4. Meßeinrichtung nach einem der vorhergehenden Ansprüche, **dadurch gekennzeichnet**, daß auf der der Meßkammer (21) abgewandten Seite des Kolbens (17) ein gesteuert mit Druckluft beaufschlagbarer Raum (29) vorgesehen ist.

5. Meßeinrichtung nach einem der vorhergehenden Ansprüche, **dadurch gekennzeichnet**, daß das Ventil (15) mit dem Kolben (17) in Wirkverbindung steht.

FIG 1

FIG 2

FIG 3